(19) Europäisches Patentamt
European Patent Office
Office européen des brevets

(11) **EP 3 977 986 A1**

(12) **EUROPEAN PATENT APPLICATION**
published in accordance with Art. 153(4) EPC

(43) Date of publication:
**06.04.2022 Bulletin 2022/14**

(21) Application number: **20815090.4**

(22) Date of filing: **21.05.2020**

(51) International Patent Classification (IPC):
***A61K 9/28*** *(2006.01)* ***A61K 31/225*** *(2006.01)*
***A61P 17/06*** *(2006.01)* ***A61P 19/02*** *(2006.01)*
***A61P 43/00*** *(2006.01)*

(52) Cooperative Patent Classification (CPC):
**A61K 9/2886; A61K 9/28; A61K 9/2846; A61K 31/225; A61P 17/06; A61P 19/02; A61P 43/00;** A61K 9/2013; Y02A 50/30

(86) International application number:
**PCT/KR2020/006647**

(87) International publication number:
**WO 2020/242132 (03.12.2020 Gazette 2020/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.05.2019 KR 20190064576**

(71) Applicant: **CURACLE Co., Ltd.**
**Seongnam-si, Gyeonggi-do 13449 (KR)**

(72) Inventors:
- **KIM, Myung-Hwa**
  **Seongnam-si Gyeonggi-do 13449 (KR)**
- **PYO, Jung-In**
  **Seongnam-si Gyeonggi-do 13449 (KR)**
- **MO, Jong Hyon**
  **Seongnam-si Gyeonggi-do 13449 (KR)**
- **LEE, Cheol Woo**
  **Seongnam-si Gyeonggi-do 13449 (KR)**
- **JI, Hyun-Ku**
  **Seongnam-si Gyeonggi-do 13449 (KR)**

(74) Representative: **Office Freylinger**
**P.O. Box 48**
**8001 Strassen (LU)**

# (54) ENTERIC TABLET CONTAINING DIMETHYL FUMARATE

(57) The present invention relates to an enteric coating tablet comprising: a core containing, as an active ingredient, dimethyl fumarate or a pharmaceutically acceptable salt thereof; and an enteric coating layer, and provides a tablet, which exhibits an effect equal to that of a capsule dosage form currently on the market, can be prepared through a simple preparation process, and is a dosage form having excellent storage stability and administration convenience, and thus can be applied to various patient groups.

FIG. 1

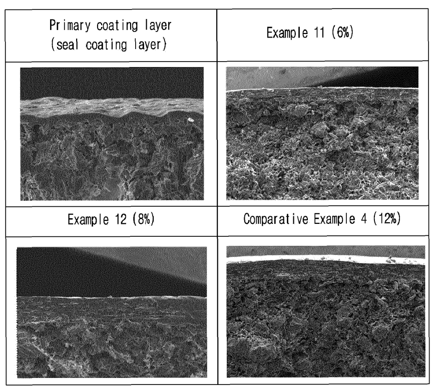


EP 3 977 986 A1

## Description

### Background of the invention

The present invention relates to a pharmaceutical preparation containing dimethyl fumarate. Particularly, the present invention relates to an enteric tablet comprising dimethyl fumarate and an enteric coating layer, and the tablet of the present invention allows dimethyl fumarate to be stably delivered to the absorption site and rapidly dissipated, so that a desired therapeutic effect can be obtained *in vivo.* The tablet of the present invention exhibits an effect equal to that of a capsule dosage form currently on the market, has advantages in terms of productivity and economy because the preparation process is simpler than that of a capsule dosage form currently on the market, and has a smaller size than the capsule, so that the patient's medication compliance can be improved. In particular, the tablet of the present invention does not contain animal-derived ingredients, so it can be used in a group of patients who are contraindicated in taking capsules due to religious issues.

Dimethyl fumarate (DMF), an active ingredient of the present invention is a compound represented by the following formula 1, which was first proposed by a German chemist in the 1950s for the treatment of psoriasis and has been used for the treatment of psoriasis for many years. In 1994, Fumaderm® (Fumapharm AG), a mixture of calcium, magnesium and zinc salts of dimethyl fumarate (DMF) and monoethyl fumarate (MEF), was approved in Germany for the treatment of psoriasis.

[Formula 1]

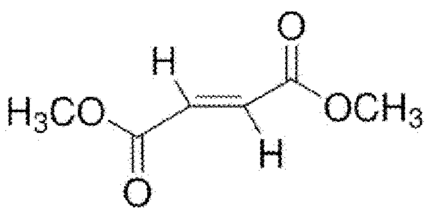

In addition to these uses for treating psoriasis, U.S. Patent No. US6,509,376 discloses that the dialkyl fumarate compound to which dimethyl fumarate belongs is useful for the treatment of autoimmune diseases such as multiple arthritis, multiple sclerosis, juvenile onset diabetes mellitus, systemic lupus erythematosus (SLE), psoriasis, psoriatic arthritis and neurodermatitis. In particular, US Patent No. US7,320,999 discloses that dimethyl fumarate is effective in multiple sclerosis. Dimethyl fumarate was first approved by FDA as a therapeutic agent for multiple sclerosis in March 2013, and is currently sold under the product name Tecfidera® in the United States and Korea. In addition, Korean Patent Publication No. 2009-0028047 discloses that dimethyl fumarate has an inhibitory effect on the proliferation of vascular smooth muscle cells, and Korean Patent No. 1379427 describes that it has an effect of preventing or treating renal fibrosis.

US6,355,676 and US6,509,376 disclose pharmaceutical compositions in the form of enteric coated micro-tablets or micro-pellets comprising dimethyl fumarate, and WO2010/126605 discloses a pharmaceutical composition comprising dimethyl fumarate in the form of a capsule containing an enteric coated micro-tablet. Currently on the market, Tecfidera® is a hard gelatin delayed-release capsule filled with micro-pellets containing dimethyl fumarate, the active ingredient.

However, the method of filling an enteric coated micro-tablet or micro-pellet in a capsule base or making a micro-tablet has a disadvantage in that the production cost increases because an additional process and manufacturing equipment are required, and there is a problem in that a loss of the main component may occur in the manufacturing process of the pellets due to the sublimation characteristic of dimethyl fumarate. In addition, since the capsule base contains an animal (cow cartilage)-derived component, there is a possibility of microbial spoilage, and there is a problem in that administration is impossible to a group of patients who are contraindicated in taking animal-derived components due to religious issues. Therefore, it is required to develop a formulation capable of solving the problems of a capsule dosage form and exhibiting an *in vivo* effect equal to that of a capsule dosage form currently on the market.

It is generally known that the enteric coating layer is coated in an amount of 10 to 12 weight% based on the total weight of the tablet core (Singh Deep Hussan et al., 2012, IOSR Journal of Pharmacy, A review on recent advances of enteric coating). Meanwhile, the present inventors identified an enteric tablet having an optimal weight ratio of the enteric coating layer with excellent bioavailability while solving the existing problems by adjusting the weight ratio of the enteric coating layer.

PRIOR ART REFERENCE

PATENT REFERENCE

US Patent No. US6,509,376
US Patent No. US7,320,999
US Patent No. US6,355,676
International Publication No. WO2010/126605
Korean Patent Publication No. 2009-0028047
Korean Patent No. 1379427

NON-PATENT REFERENCE

Singh Deep Hussan et al., 2012.

**Brief summary of the invention**

Accordingly, the present inventors have studied to solve the above problems, and as a result, the present inventors have completed the present invention by confirming that when the enteric coating layer surrounding the core containing dimethyl fumarate is used in an optimal amount, the problems of a capsule dosage form such as sublimation of active ingredients and complicated manufacturing processes can be solved, and a tablet formulation having excellent bioavailability *in vivo* can be prepared.

It is an object of the present invention to provide an enteric tablet for preventing or treating inflammatory or autoimmune diseases or disorders, diseases caused by proliferation of vascular smooth muscle cells, renal fibrosis, and the like. Specifically, it is an object of the present invention to provide an enteric tablet comprising dimethyl fumarate as an active ingredient, and containing an enteric coating layer in an amount of 6 to 9 weight% based on the weight of the core containing the active ingredient, so that dimethyl fumarate can be stably delivered to the absorption site and quickly dissipated, and a desired therapeutic effect can be expected *in vivo.*

In addition, another object of the present invention is to provide a formulation that has excellent storage stability, administration convenience, various applicable patient groups, and a bioavailability equivalent to that of a commercially available capsule formulation while requiring a low production cost due to a simple preparation process.

To achieve the above objects, in an aspect of the present invention, the present invention provides an enteric coating tablet comprising a core containing dimethyl fumarate or a pharmaceutically acceptable salt thereof as an active ingredient; and an enteric coating layer, wherein the enteric coating layer is included in an amount of 6 to 9 weight parts based on 100 weight parts of the core.

In another aspect of the present invention, the present invention provides a method for preparing an enteric coating tablet comprising the following steps:

a step of preparing a mixture by mixing dimethyl fumarate or a pharmaceutically acceptable salt thereof, and a pharmaceutically acceptable additive;
a step of preparing a core by direct-tableting the mixture; and
a step of enteric coating the core.
wherein, the enteric coating is performed with 6 to 9 weight parts of the enteric coating layer based on 100 weight parts of the core.

The enteric tablet according to the present invention exhibits a preventive or therapeutic effect on inflammatory or autoimmune diseases or disorders, diseases caused by proliferation of vascular smooth muscle cells, renal fibrosis, and the like. More particularly, it is possible to provide a tablet, a dosage form that has excellent storage stability, administration convenience, and can be applied to various patient groups, through a simple preparation process without loss of active ingredients that may occur during a micro-pellet preparation process. In particular, the enteric tablet of the present invention can secure a drug release pattern equivalent to that of a commercially available capsule formulation *in vivo,* thereby exhibiting excellent bioavailability.

**Brief description of the drawings**

Figure 1 is a diagram illustrating the results of observation through a scanning electron microscope to confirm the thickness of the primary coating layer (seal coating layer) and the coating layers according to Examples 11 and 12, and Comparative Example 4.

Figure 2 is a graph illustrating the results of confirming the elution rates of the enteric coating tablets according to Examples 1 to 3 in order to compare and analyze the elution rate of the tablet according to the enteric coating ratio.

Figure 3 is a graph illustrating the results of confirming the elution rates of the enteric coating tablets according to Examples 2 and 4 in order to compare and analyze the elution rate of the tablet according to the copolymer ratio of the enteric coating base.

Figure 4 is a graph showing the results of confirming the elution rates of the enteric coating tablets according to Examples 4 and 5 in order to compare and analyze the elution rate of the tablet according to the coating ratio of the enteric coating base.

Figure 5 is a graph illustrating the results of evaluating the weight loss rate (%) of dimethyl fumarate (main ingredient) and a mixture containing dimethyl fumarate and a pharmaceutically acceptable additive in order to establish an appropriate temperature range for the drying step by evaluating the weight loss rate in the coating process.

Figure 6 is a graph illustrating the results of confirming the elution rates of the enteric coating tablets according to Examples 5 and 6 in order to evaluate the elution rate according to the particle size of dimethyl fumarate.

Figure 7 is a graph illustrating the results of confirming the elution rates of the formulations according to Examples 5 and 7, and Comparative Example 1 in order to evaluate the elution rate according to the use of an alkalizing agent.

Figure 8 is a graph illustrating the results of confirming the elution rates of the formulations according to Example 5 and Comparative Example 1 under the condition of pH 1.2 solution (artificial gastric juice condition, disintegrating solution 1, The Korean Pharmacopoeia) in order to evaluate the elution rate of the formulation containing 120 mg of dimethyl fumarate.

Figure 9 is a graph illustrating the results of confirming the elution rates of the formulations according to Example 5 and Comparative Example 1 under the condition of pH 6.8 solution (artificial intestinal juice) in order to evaluate the elution rate of the formulation containing 120 mg of dimethyl fumarate.

Figure 10 is a graph illustrating the results of confirming the elution rates of the enteric coating tablets according to Examples 5 and 8 in order to confirm the elution rate of the tablet containing 240 mg of dimethyl fumarate.

Figure 11 is a graph illustrating the results of confirming the *in vivo* kinetics of the drug by orally administering the formulations according to Example 11 and Comparative Examples 1 to 4 to beagle dogs for pharmacokinetic evaluation of the enteric coating tablet according to the present invention.

**Detailed description of the invention**

Hereinafter, the present invention is described in detail.

In an aspect of the present invention, the present invention provides an enteric coating tablet comprising a core containing dimethyl fumarate or a pharmaceutically acceptable salt thereof as an active ingredient; and an enteric coating layer, wherein the enteric coating layer is included in an amount of 6 to 9 weight parts based on 100 weight parts of the core.

In the case of commercially available capsule formulations, loss of dimethyl fumarate may occur during the preparation process, and there are problem in that administration is impossible to a group of patients who are contraindicated in taking animal-derived components due to religious issues, administration convenience, and the like. On the other hand, the enteric coating tablet of the present invention is made on the basis that dimethyl fumarate is stably delivered to the absorption site and rapidly dissipated to exhibit a therapeutic effect by adjusting the content of the enteric coating layer. In particular, the enteric coating layer is typically used in an amount of 10 to 12 weight% or 10 to 13 weight% relative to the total weight of the tablet core. In the present invention, by using 6 to 9 weight parts based on 100 weight parts of the tablet core containing dimethyl fumarate or a pharmaceutically acceptable salt thereof, dissolution proceeds rapidly at the absorption site, thereby ensuring excellent bioavailability.

At this time, the active ingredient can be included in an amount of 20 to 60 weight%, preferably 25 to 55 weight%, 30 to 50 weight%, 35 to 45 weight%, 40 to 45 weight%, 43 to 45 weight%, or about 44 weight% based on the core.

In addition, the active ingredient can be included in the core in an amount of 50 mg to 500 mg, preferably 60 mg to 480 mg, 100 mg to 400 mg, 50 mg to 400 mg, 100 mg to 350 mg, 100 mg to 300 mg, 100 mg to 250 mg, 100 mg to 150 mg, 200 mg to 250 mg, 330 mg to 400 mg, 330 mg to 480 mg, 50 mg to 100 mg, about 60 mg, about 120 mg, about 240 mg, about 360 mg, about 480 mg, more preferably 60 mg, 120 mg, 240 mg, 360 mg or 480 mg. Dose-proportional linear elimination kinetics of dimethyl fumarate or a pharmaceutically acceptable salt thereof has demonstrated from 120 mg to 360 mg.

The core includes one or more pharmaceutically acceptable additives selected from the group consisting of excipients, disintegrants and lubricants. The pharmaceutically acceptable additives are not limited to the excipients, disintegrants, and lubricants, and can be used as long as they are pharmaceutically commonly used additives. For example, additives such as excipients, binders, disintegrants, antioxidants, surfactants, lubricants, plasticizers, and

pigments can be included.

Examples of the excipient include starch, lactose, anhydrous lactose, microcrystalline cellulose, silicified microcrystalline cellulose, hypromellose, silicic anhydride, calcium phosphate, anhydrous calcium phosphate, calcium hydrogen phosphate, anhydrous calcium hydrogen phosphate, calcium silicate, dextrin, dextrose, dextrate, mannitol, maltose, sorbitol, sucrose, polyethylene glycol, sodium chloride, and the like, and these can be used alone or in combination of two or more. Preferably, silicified microcrystalline cellulose can be used.

The disintegrant can include crospovidone, croscarmellose sodium, sodium glycolate starch, pregelatinized starch, low-substituted hydroxypropyl cellulose, grain starch, and the like, and these can be used alone or in combination of two or more. Preferably, croscarmellose sodium can be used.

Examples of the lubricant include magnesium stearate, stearic acid, talc, silicon dioxide, colloidal silicon dioxide, sodium stearyl fumarate, sodium lauryl sulfate, poloxamer, and the like, and these can be used alone or in combination of two or more. Preferably, colloidal silicon dioxide or magnesium stearate can be used, and most preferably, colloidal silicon dioxide and magnesium stearate can be used.

Examples of the plasticizer include triethyl citrate, acetyl tributyl citrate, glycerol acetic acid fatty acid ester, triacetin, dibutyl phthalate, polysorbate 80, polyethylene glycol, propylene glycol, and the like, and these can be used alone or in combination of two or more.

Examples of the binder include povidone, copovidone, methyl cellulose, hydroxymethyl cellulose, hydroxypropyl methyl cellulose, hydroxypropyl cellulose, hydroxyethyl cellulose, gelatin, guar gum, xanthan gum, and the like, and these can be used alone or in combination of two or more.

Examples of the antioxidant include dibutylhydroxy toluene, butylhydroxy toluene, butylhydroxy anisole, tert-butylhydroquinone, propyl gallate, vitamin C, and the like, and these can be used alone or in combination of two or more.

Examples of the surfactant include sodium lauryl sulfate, sodium stearate, polysorbate 80, poloxamer, and the like, and these can be used alone or in combination of two or more.

A seal-coating layer can be further included between the core and the enteric coating layer. At this time, the seal-coating layer is also referred to as an intermediate coating layer, a primary coating layer, or a non-enteric coating layer. The seal-coating layer can include a cellulose-based polymer, preferably hydroxypropyl methyl cellulose, but not always limited thereto, and is not particularly limited as long as it is a non-enteric coating base. The cellulose-based polymer can be at least one selected from the group consisting of polyvinyl alcohol (PVA), polyethylene glycol, polyvinyl alcohol-polyethylene glycol graft copolymer (eg Kollicoat-IR), ethyl cellulose, hydroxypropyl cellulose (HPC), lactose and mannitol. The seal-coating layer can be included in an amount of 1 to 3 weight parts based on 100 weight parts of the core, preferably 1 to 2 weight parts, about 1.5 weight parts, or about 2 weight parts.

The core can further include an alkalizing agent, wherein the weight ratio of the active ingredient and the alkalizing agent may be 12:0.5 to 12:2, 12:0.7 to 12:1.8, 12:0.8 to 12:1.5, 12:0.9 to 12:1.3, or 12:0.9 to 12:1.1, and preferably can be 12:1.

The alkalizing agent can be included in an amount of 2 to 5 weight%, 2.5 to 4.5 weight%, 3 to 4 weight%, 3.5 to 4 weight%, or about 3.7 weight% based on the core.

As the alkalizing agent, a known alkalizing agent can be used in order to increase the aqueous solubility of the active ingredient. Preferably, meglumine or a pharmaceutically acceptable salt thereof can be used as the alkalizing agent to improve compression moldability, adsorption, disintegration, stability, etc. suitable for tablets.

For the enteric coating layer, one or more enteric coating polymers selected from the group consisting of enteric acrylic acid-based copolymers selected from the group consisting of styrene acrylic acid copolymer, ethyl methacrylate copolymer, methyl acrylate octyl methacrylate copolymer and ethyl methacrylate acrylate copolymer; enteric cellulose-based polymers selected from the group consisting of hydroxypropyl methyl cellulose acetate succinate, hydroxypropyl methyl cellulose phthalate, hydroxymethyl ethyl cellulose phthalate, cellulose acetate phthalate, cellulose acetate maleate, cellulose acetate succinate, cellulose acetate maleate, cellulose benzoate phthalate, cellulose propionate phthalate, methyl cellulose phthalate, carboxymethyl ethyl cellulose, ethylhydroxy ethyl cellulose phthalate, carboxymethyl ethyl cellulose and ethyl hydroxyethyl cellulose phthalate; enteric maleic acid-based copolymers selected from the group consisting of vinyl acetate maleic acid anhydride copolymer, styrene maleic acid anhydride copolymer, styrene maleic acid monoesterol copolymer, vinyl methyl ether maleic acid anhydride copolymer, ethylene maleic acid anhydride copolymer, vinyl butyl ether maleic acid anhydride copolymer, acrylonitrile methyl acrylate maleic acid anhydride copolymer and butyl acrylate styrene maleic acid anhydride copolymer; and enteric polyvinyl-based polymers selected from the group consisting of polyvinyl alcohol phthalate, polyvinyl acetal phthalate, polyvinyl butyrate phthalate and polyvinyl acetacetal phthalate; can be used, but the enteric coating polymer is not particularly limited as long as it is a pharmaceutically acceptable enteric coating base. The enteric tablet according to the present invention can solve the difference in quality between batches due to the non-uniformity of mixing that may occur when two or more types of coating bases are mixed by mixing the additives other than the enteric coating base.

An enteric coating layer can be formed using an enteric coating base comprising the enteric coating polymer in an amount of 20 to 80 wt%. At this time, the polymer included in the enteric coating base can be included in an amount

of 20 to 60 weight%, 40 to 80 weight%, 40 to 60 weight%, 35 to 45 weight%, 55 to 65 weight%, about 40 weight%, or about 60 weight%.

When the enteric coating layer is 5 weight parts or less based on 100 weight parts of the core, there may be a problem in that the drug is eluted and decomposed in the stomach. On the other hand, when the enteric coating layer is 9 weight parts or more based on 100 weight parts of the core, the absorption rate of the drug in the body is lowered, and it takes a long time to reach the effective concentration, which may cause a problem that the therapeutic effect cannot be properly exhibited. The content range of the enteric coating layer according to the present invention is preferable to control the elution rate so that the drug dimethyl fumarate or a pharmaceutically acceptable salt thereof is stably delivered to the absorption site *in vivo* and dissolution is possible so that the therapeutic effect can be sufficiently exhibited.

The particle size distribution of dimethyl fumarate or a pharmaceutically acceptable salt thereof is that (a) the mean particle size of the lower 90% of the particles (D90) is 100 μm or less; (b) the mean particle size of the lower 50% of the particles (D50) is 50 μm or less; and (c) the mean particle size of the lower 10% of the particles (D10) is 20 μm or less, (a) the mean particle size of the lower 90% of the particles (D90) is 80 μm or less; (b) the mean particle size of the lower 50% of the particles (D50) is 40 μm or less; and (c) the mean particle size of the lower 10% of the particles (D10) is 15 μm or less, or (a) the mean particle size of the lower 90% of the particles (D90) is 50 μm or less; (b) the mean particle size of the lower 50% of the particles (D50) is 30 μm or less; and (c) the mean particle size of the lower 10% of the particles (D10) is 10 μm or less.

The thickness of the coating layer of the enteric coating tablet can be 20 μm to 90 μm, 30 μm to 80 μm, 30 μm to 50 μm, 60 μm to 80 μm, 35 μm to 50 μm, 65 μm to 80 μm, 35 μm to 80 μm, or 40 μm to 75 μm. At this time, the thickness of the coating layer of the enteric coating tablet can be the thickness of the enteric coating layer, or the thickness of the coating layer including the seal-coating layer and the enteric coating layer.

The enteric coating tablet can be prepared by a conventional tablet manufacturing method such as a conventional dry/wet granulation method, a direct powder compression method or a direct compression method, and preferably can be prepared by a direct compression method.

The enteric coating tablet can be used for the prevention or treatment of organ fibrosis, neurodegenerative disease, psoriasis, polyarthritis, juvenile diabetes, Hashimoto's disease, Grave's disease, systemic lupus erythematosus, Sjogren's syndrome, pernicious anemia, chronic active hepatitis, lupus-like hepatitis, rheumatoid arthritis, autoimmune disease, inflammatory disease, diseases caused by proliferation of vascular smooth muscle cells or optic neuritis. At this time, the organ fibrosis is at least one selected from the group consisting of renal fibrosis, cardiac fibrosis, pancreatic fibrosis, lung fibrosis, vascular fibrosis, skin fibrosis, bone marrow fibrosis, liver fibrosis, scleroderma, cystic fibrosis, pancreatic fibrosis and intestinal fibrosis; the renal fibrosis is at least one selected from the group consisting of renal failure, diabetic nephropathy, glomerulosclerosis, renal tubular fibrosis, glomerulonephritis, chronic renal failure, acute renal injury, chronic kidney disease, end-stage renal disease and albuminuria; the liver fibrosis is at least one selected from the group consisting of cirrhosis, hepatic nephropathy, hepatic purpura, metabolic liver disease, chronic liver disease, hepatitis B virus infection, hepatitis C virus infection, hepatitis D virus infection, schistosomiasis, alcoholic liver disease, non-alcoholic fat hepatitis, obesity, diabetes, protein deficiency, coronary artery disease, auto-immune hepatitis, cystic fibrosis, alpha-1 antitrypsin deficiency and primary biliary cirrhosis; the lung fibrosis is at least one selected from the group consisting of bronchitis, acute bronchitis, diffuse panbronchiolitis (DPB), bronchiolitis, idiopathic pulmonary fibrosis (IPF), acute interstitial pneumonia, lung transplantation, radiation-induced pulmonary fibrosis, acute respiratory distress syndrome (ARDS), chronic obstructive pulmonary disease (COPD), asthma, bronchiectasis, pulmonary tuberculosis, pneumonia, pneumoconiosis, hypersensitivity pneumonia, pulmonary edema and sarcoidosis; the skin fibrosis is at least one selected from the group consisting of scarring, hypertrophic scarring, keloid scarring, cutaneous fibrosis disorder, wound healing, delayed wound healing, psoriasis and scleroderma; and the neurodegenerative diseases is at least one selected from the group consisting of multiple sclerosis, systemic sclerosis, amyotrophic lateral sclerosis, Parkinson's disease, Huntington's disease, Alzheimer's disease, acute transverse myelitis, acute disseminated encephalomyelitis, optic neuritis, acute necrotizing retinitis, transverse myelitis, chronic progressive myelopathy, progressive multifocal leukoencephalopathy, radiation myelopathy, central pontine myelinolysis, leukodystrophy, chronic inflammatory demyelinating polyneuropathy (CIDP) and acute inflammatory demyelinating polyneuropathy (AIDP). However, these are only examples and the indications of the enteric coating tablet are not necessarily limited thereto.

The enteric coating tablet can include powder form, and is preferably prepared as an enteric coating tablet in solid form, but it is not impossible to manufacture in liquid form, and this is not excluded from the scope of rights.

The enteric coating tablet can be administered as an individual therapeutic agent or may be administered in combination with other therapeutic agents, can be administered sequentially or simultaneously with the conventional therapeutic agents, and can be administered singly or in multiple.

The term "administration" used in this specification means introducing the enteric tablet into a patient by any suitable method. The enteric tablet can be administered through various routes, either oral or parenteral, as long as it can reach the target tissue. Preferably, the enteric tablet can be administered orally. In addition, the enteric tablet can be prepared in various dosage forms depending on the desired administration method.

The administration frequency of the enteric coating tablet is not particularly limited, but can be administered once or twice a day, or can be administered several times by dividing the dose. For example, a 120 mg tablet can be administered as one tablet each in the morning and afternoon, or a 240 mg tablet can be administered as one tablet in the morning or afternoon. The subject to be administered can be any animal including humans, and the animal can be a mammal, such as cattle, horses, sheep, pigs, goats, camels, antelopes, dogs, cats, and the like, but not always limited thereto.

In another aspect of the present invention, the present invention provides a method for preparing an enteric coating tablet comprising the following steps:

a step of preparing a mixture by mixing dimethyl fumarate or a pharmaceutically acceptable salt thereof, and a pharmaceutically acceptable additive;
a step of preparing a core by directly tableting the mixture; and
a step of enteric coating the core.

At this time, the enteric coating is performed with 6 to 9 weight parts of the enteric coating layer based on 100 weight parts of the core.

The method can further include a step of seal-coating before the step of enteric coating.

At this time, the enteric coating step and/or the seal-coating step can be conducted at 20°C to 50°C, 20°C to 40°C, and preferably at about 25°C to 35°C.

The enteric coating layer can additionally include additives such as excipients, binders, disintegrants, antioxidants, surfactants, lubricants, plasticizers, and pigments.

The step of enteric coating can be performed with a coating solution in which an enteric coating base and/or a pharmaceutically acceptable additive are dissolved in a solvent. The solvent can be used as one or a combination of two or more selected from the group consisting of purified water, alcohol, alkyl acetate, dimethyl formamide, dimethyl sulfoxide, acetone, anisole, acetic acid, butylmethyl ether, ethyl ether, ethyl formate, formic acid, pentane, heptane, methylethyl ketone and methylisobutyl ketone.

The coating can be carried out through known means. For example, in the case of spray coating, a pan coating device, a drum coating device, a fluidized bed coating device, or an agitated fluidized bed coating device can be used. As a sprayer attached to such a device, an air sprayer, an airless sprayer or a 3-fluid sprayer can be used. In the case of the dry type, for example, a centrifugal fluidized coating device, a pan coating device, a fluidized bed coating device, a centrifugal motorized fluidized bed coating device, and the like can be used.

With respect to the preparation method of the enteric coating tablet, the above-described content for the enteric coating tablet can be applied.

Hereinafter, the present invention will be described in detail by the following examples and experimental examples.

However, the following examples and experimental examples are only for illustrating the present invention, and the contents of the present invention are not limited thereto.

Example: Preparation of enteric coating tablet

Table 1

| | | Compone nt | Dose (mg/tablet) | | | | | | |
|---|---|---|---|---|---|---|---|---|---|
| | | | Examp le 1 | Examp le 2 | Examp le 3 | Examp le 4 | Examp le 5 | Examp le 6 | Examp le 7 |
| Cor e | Main comp onen t | Dimethy 1 fumarat e | 120.0 | 120.0 | 120.0 | 120.0 | 120.0 | 120.0 | 120.0 |
| | Alka lini zing agen t | Meglumi ne | 10.0 | 10.0 | 10.0 | 10.0 | 10.0 | 10.0 | - |
| | Excipient (silicified microcrystall ine cellulose), disintegrant (croscarmello se sodium and/or crospovidone), , lubricant (colloidal silicon dioxide and/or magnesium stearate) | | 140.0 | 140.0 | 140.0 | 140.0 | 140.0 | 140.0 | 140.0 |
| Uncoated tablet | | | 270.0 | 270.0 | 270.0 | 270.0 | 270.0 | 270.0 | 260.0 |
| Primary coating | | OPADRY 03K19229 | 5.4 | 5.4 | 5.4 | 5.4 | 5.4 | 5.4 | 5.2 |
| Secondar y coating | | ACRYL-EZE MP 93018508 | 10.8 | 16.2 | 21.6 | - | - | - | - |
| | | ACRYL-EZE MP 93018509 | - | - | - | 16.2 | 21.6 | 21.6 | 20.8 |

Table 2

| | | Componen t | Dose (mg/tablet) | | | | |
|---|---|---|---|---|---|---|---|
| | | | Example 8 | Example 9 | Example 10 | Example 11 | Example 12 |
| Core | Main component | Dimethyl fumarate | 240.0 | 240.0 | 240.0 | 120.0 | 120.0 |
| | Alkalini zing agent | meglumine | 20.0 | - | - | - | - |
| | Excipient (silicified microcrystalline cellulose), disintegrant (croscarmellose sodium and/or crospovidone), , lubricant (colloidal silicon dioxide and/or magnesium stearate) | | 280.0 | 300.0 | 300.0 | 150.0 | 150.0 |
| Uncoated tablet | | | 540.0 | 540.0 | 540.0 | 270.0 | 270.0 |
| Primary coating | OPADRY 03K19229 | | 10.8 | 8.0 | 8.0 | 4.0 | 4.0 |
| Secondary coating | ACRYL-EZE MP 93018508 | | - | 43.2 | 54.0 | 16.2 | 22.0 |
| | ACRYL-EZE MP 93018509 | | 43.2 | - | - | - | - |

**Preparation of enteric coating tablet containing dimethyl fumarate**

The angle of repose of the mixture containing dimethyl fumarate is 40° or less, and it is usually evaluated that the fluidity is good enough to allow direct tableting if the angle of repose is less than 40°. On the other hand, when the

wet granulation method is applied to improve the fluidity, there is a concern about loss due to sublimation of dimethyl fumarate caused by the use of a solvent and drying. Therefore, an enteric coating tablet comprising dimethyl fumarate was prepared as follows by minimizing contact with water and applying a direct tableting method with a simple preparation process.

According to the compositions of Tables 1 and 2, the enteric coating tablets according to Examples 1 to 12 were prepared through the following steps:

preparing a core (uncoated tablet, that is, a tablet in a compressed state without coating) by mixing dimethyl fumarate and pharmaceutically acceptable additives (excipients (silicified microcrystalline cellulose), disintegrants (croscarmellose sodium and/or crospovidone), lubricants (colloidal silicon dioxide and/or magnesium stearate) and alkalinizing agents (meglumine)), and compressing the mixture;
primary coating (seal-coating) the core with a coating solution in which a non-enteric coating base is dissolved in a solvent; and
secondary coating the core with a coating solution in which an enteric coating base is dissolved in a solvent.

When the seal-coating (primary coating) is applied before the enteric coating, there is an advantage in that the adhesion to the tablet surface of the enteric coating base can be increased and the acid resistance can be increased. At this time, a polyvinylalcohol (PVA) base can be used as the seal-coating base, but when using the PVA base, the polymer ratio in the coating base is low, so it should be coated with about 6 to 10% of the weight of the uncoated tablet thicker than the HPMC base. In addition, in this case, there is a high possibility that the enteric coating film is not uniformly applied depending on the surface and curve of the tablet, so that the acid resistance is highly likely to be impaired. The PVA base should be coated with water-based coating using water as a solvent and dried for a long time at a high temperature of 45°C or higher. Therefore, water-based coating is not an appropriate coating method.

On the other hand, in case of seal-coating with a hydroxypropyl methylcellulose (HPMC) base, it is possible to coat thinly with a coating ratio of about 1.5 to 3% of the weight of the uncoated tablet, and oil-based coating using an organic solvent of ethanol is possible. The loss of dimethyl fumarate can be minimized by drying for a short time at a low temperature of about 25 ~ 35°C. In addition, when using the HPMC base, there is an advantage that the enteric coating film is stably maintained while the enteric coating base containing copolymer is well adhered to the surface of the seal-coating film. Therefore, in the enteric coating tablet containing dimethyl fumarate according to Example, OPADRY 03K19229 mainly composed of HPMC was used as a seal-coating base.

ACRYL-EZE MP, which is an enteric coating base, is classified into ACRYL-EZE MP 93018508 and ACRYL-EZE MP 93018509 according to the composition ratio of methacrylic acid and ethyl acrylate copolymer. As shown in Table 3, when the weight ratio of methacrylic acid and ethyl acrylate is 60 w/w%, it is classified as ACRYL-EZE MP 93018508, and when the weight ratio of methacrylic acid and ethyl acrylate is 40 w/w%, it is classified as ACRYL-EZE MP 93018509. As an enteric coating base, there is also a hydroxypropyl methylcellulose phthalate-based coating base in addition to the methacrylic acid and ethyl acrylate copolymer. On the other hand, the hydroxypropyl methylcellulose phthalate-based coating base has a high organic solvent usage, so it is highly possible to detect residual solvent, and the coating time is also longer than that of the methacrylic acid and ethyl acrylate copolymer-based coating base, so it is generally not suitable for use.

Table 3

| Component | w/w% | Brand name |
|---|---|---|
| Methacrylic acid and ethyl acrylate copolymer | 60 | ACRYL-EZE MP 93018508 |
| | 40 | ACRYL-EZE MP 93018509 |

**<Comparative Example>**

In Comparative Example 1, 120 mg of the commercially available reference drug Tekpidera capsule (Tecfidera®, Eisai Korea Inc.) was used. In Comparative Example 2 to Comparative Example 4, tablets were prepared in the same manner as described in Example according to the ingredient table of Table 4.

Table 4

| Component | | | Comparative Example 1 | Dose (mg/tablet) | | |
|---|---|---|---|---|---|---|
| | | | | Comparative Example 2 | Comparative Example 3 | Comparative Example 4 |
| Co re | Main compo nent | Dimethyl fumarate | 120.0 | 120.0 | 120.0 | 120.0 |
| | Alkal inizi ng agent | Meglumine | - | - | - | - |
| | Excipient (silicified microcrystalline cellulose), disintegrant (croscarmellose sodium and/or crospovidone), lubricant (colloidal silicon dioxide and/or magnesium stearate) | | Proper amount | 150.0 | 150.0 | 150.0 |
| Uncoated tablet | | | | 270.0 | 270.0 | 270.0 |
| Primary coating | OPADRY 03K19229 | | - | 4.0 | 4.0 | 4.0 |
| Seconda ry coating | ACRYL-EZE MP 93018508 | | - | 13.5 | 28.0 | 33.0 |
| | ACRYL-EZE MP 93018509 | | - | - | - | - |

**<Experimental Example 1> Measurement of coating layer thickness**

To measure the thickness of the enteric coating layer of the enteric-coated tablets according to Example 11, Example 12 and Comparative Example 4, the primary coating layer (seal-coating), the coating layers of the tablets of Example 11, Example 12 and Comparative Example 4 were observed under scanning electron microscope (SEM) using ESEM (Thermo Fisher, Quattro S). At this time, the weight of the enteric coating layer (secondary coating layer) of the tablet of Example 11 was 6% based on the total weight of the core, 8% in Example 12, and 12% in Comparative Example 4. For SEM observation, the primary coating layer (seal-coating), the coating layers of the tablets of Example 11, Example 12 and Comparative Example 4 were pretreated by depositing Os as thin as 10 nm or less using an Os coater. The results are shown in Table 5 and Figure 1.

Table 5

| | Primary coating layer (seal-coating) | Example 11 | Example 12 | Comparative Example 4 |
|---|---|---|---|---|
| Mean measured value (μm) | 17±6 | 43±6 | 71±10 | 109±6 |

In the results of Table 5, the film thickness of the enteric coating layer of Examples 11, 12, and Comparative Example 4 is the value obtained by subtracting the thickness of the primary coating layer (seal-coating layer) from each mean measured value. As shown in the above results, the thickness of the enteric coating layer of Example 11 was the thinnest, followed by those of Example 12 and Comparative Example 4. That is, the enteric coating layer was thinner as the weight ratio to the total weight of the core was lower. It was confirmed that the elution rate evaluation and pharmacokinetic results were affected by the thickness of the coating layer. Therefore, it was confirmed that the elution rate evaluation and pharmacokinetic results were affected according to the thickness of the coating layer.

**<Experimental Example 2> Evaluation of elution rate according to enteric coating ratio**

**2-1. Elution rate of tablets at pH 6.8 according to enteric coating ratio**

In order to evaluate the elution rate of the tablet according to the enteric coating ratio, the elution rate of the enteric coating tablets according to Examples 1 to 3 in pH 6.8 solution was evaluated. The tablets of Examples 1 to 3 contained 10.8 mg/tablet, 16.2 mg/tablet, and 21.6 mg/tablet of ACRYL-EZE MP 93018508 (methacrylic acid and ethyl acrylate copolymer 60% w/w) as an enteric coating base, respectively.

To evaluate the elution rate, a buffer solution of pH 6.8 (Mcilvane buffer) was prepared, and a dissolution test was performed on each eluate according to the second method (paddle method). Particularly, the buffer solution was maintained at 900 mL, the stirring speed was maintained at 75 rpm, and the temperature of the buffer solution was maintained at 37 ± 0.5°C. After the start of the dissolution test during the test, the final time point was set based on the general time to stay in the internal organ (intestine) representing pH 6.8, and the sample solution was collected by setting the intermediate time point at regular intervals. The collected sample solution was filtered through a filter and analyzed by high performance liquid chromatography (HPLC). The results are shown in Table 6 and Figure 2.

Table 6

| Elution rate (%) | | | |
|---|---|---|---|
| **Min.** | **Example 1** | **Example 2** | **Example 3** |
| 0 | 0.0 | 0.0 | 0.0 |
| 5 | 28.0 | 18.2 | 2.3 |
| 10 | 86.1 | 88.0 | 55.6 |
| 15 | 89.0 | 90.9 | 88.0 |
| 30 | 89.5 | 89.4 | 89.6 |
| 45 | 87.4 | 87.8 | 91.0 |
| 60 | 87.6 | 87.1 | 90.3 |
| 90 | 84.0 | 85.1 | 87.4 |
| 120 | 83.3 | 82.7 | 85.6 |

As shown in Table 6 and Figure 2, it was confirmed that the higher the enteric coating ratio, the delayed the initial elution rate of the tablet. That is, the dissolution of the tablet according to Example 1 containing 10.8 mg/tablet of ACRYL-EZE MP 93018508 (methacrylic acid and ethyl acrylate copolymer 60%w/w) as an enteric coating base proceeded most rapidly.

**2-2. Elution rate of tablets at pH 6.8 according to copolymer ratio of enteric coating base**

In order to evaluate the elution rate of the tablet according to the copolymer ratio of the enteric coating base, the elution rate at pH 6.8 of the enteric coating tablets according to Examples 2 and 4 was measured. The tablet of Example 2 contained 16.2 mg/tablet of ACRYL-EZE MP 93018508 (methacrylic acid and ethyl acrylate copolymer 60% w/w) as an enteric coating base, and the tablet of Example 4 contained 16.2 mg/tablet of ACRYL-EZE MP 93018509 (methacrylic acid and ethyl acrylate copolymer 40% w/w) as an enteric coating base.

The elution rate evaluation was performed in the same manner as described in Example 2-1, and the results are shown in Table 7 and Figure 3.

Table 7

| Elution rate (%) | | |
|---|---|---|
| **Min.** | **Example 2** | **Example 4** |
| 0 | 0.0 | 0.0 |
| 5 | 18.2 | 80.1 |
| 10 | 88.0 | 90.5 |
| 15 | 90.9 | 92.4 |
| 30 | 89.4 | 93.5 |
| 45 | 87.8 | 93.9 |
| 60 | 87.1 | 93.9 |
| 90 | 85.1 | 92.7 |

(continued)

| Elution rate (%) | | |
|---|---|---|
| Min. | Example 2 | Example 4 |
| 120 | 82.7 | 91.6 |

As shown in Table 7 and Figure 3, it was confirmed that the initial elution rate of the tablet of Example 2 containing ACRYL-EZE MP 93018508 having a high composition ratio of methacrylic acid and ethyl acrylate copolymer as an enteric coating base was relatively delayed. That is, the tablets of Example 2 and Example 4 contained both 16.2 mg/tablet of the enteric coating base, while the dissolution of the enteric coating tablet of Example 4 containing ACRYL-EZE MP 93018509 having a relatively low composition ratio of methacrylic acid and ethyl acrylate copolymer (40% w/w) proceeded better.

**2-3. Elution rate of tablets at pH 6.8 according to coating ratio of the enteric coating base ACRYL-EZE MP 93018509**

In order to evaluate the elution rate of the tablet according to the coating ratio of the enteric coating base ACRYL-EZE MP 93018509, which was confirmed to have relatively better dissolution through Example 2-2, the elution rate at pH 6.8 of the enteric coating tablets according to Examples 4 and 5 was measured. The tablets of Examples 4 and 5 contained 16.2 mg/tablet, and 21.6 mg/tablet of ACRYL-EZE MP 93018509 (methacrylic acid and ethyl acrylate copolymer 40% w/w) as an enteric coating base, respectively.

The elution rate evaluation was performed in the same manner as described in Example 2-1, and the results are shown in Table 8 and Figure 4.

Table 8

| Elution rate (%) | | |
|---|---|---|
| Min. | Example 4 | Example 5 |
| 0 | 0.0 | 0.0 |
| 5 | 80.1 | 83.3 |
| 10 | 90.5 | 93.8 |
| 15 | 92.4 | 94.9 |
| 30 | 93.5 | 95.1 |
| 45 | 93.9 | 94.8 |
| 60 | 93.9 | 94.6 |
| 90 | 92.7 | 93.5 |
| 120 | 91.6 | 92.2 |

As shown in Table 8 and Figure 4, it was confirmed that the elution rate of the tablet of Example 4 with an enteric coating ratio of 6% and the tablet of Example 5 with an enteric coating ratio of 8% showed a similar trend. However, in consideration of the stability of the tablet properties, it is preferable to set the coating ratio to 8%. Therefore, through the results of 2-1 to 2-3, it was confirmed that the tablet of Example 5 in which the coating ratio of the enteric coating base ACRYL-EZE MP 93018509 was 8% was the most optimal enteric coating tablet.

**<Experimental Example 3> Setting temperature range by evaluating weight loss rate in coating process**

Dimethyl fumarate has a property of being lost by sublimation depending on the storage temperature. Accordingly, in order to confirm the degree of weight loss according to the coating drying temperature, the degree of weight loss was repeatedly evaluated for 2 weeks at a temperature of 60°C for dimethyl fumarate and a mixture thereof. At this time, the mixture of dimethyl fumarate was a mixture in which dimethyl fumarate and other pharmaceutically acceptable additives were mixed. The results are shown in Figure 5.

As shown in Figure 5, dimethyl fumarate (main component) and a mixture thereof continued to lose weight during drying and sublimation during the coating process. Therefore, it is necessary to lower the supply air temperature

to prevent the loss of dimethyl fumarate. At this time, when the supply air temperature during the coating process was about 55 to 60°C, the product temperature was about 35 to 40°C, so stable and fast drying could be achieved during seal-coating and enteric coating, while the temperature was too low to dry the coating. Therefore, the drying temperature of the product was appropriate in the temperature range of about 25 to 35°C.

**<Experimental Example 4> Evaluation of elution rate according to particle size of dimethyl fumarate**

The degree of solubilization of a poorly soluble drug increases as the particle size of the drug increases according to "Noyes-Whitney equation", and thus the solubility of the drug tends to improve. Therefore, the particle size of dimethyl fumarate was adjusted under the conditions shown in Table 9, and the comparative elution patterns of the tablet of Example 5 containing micronized dimethyl fumarate (that is, dimethyl fumarate finely pulverized to D90 100 μm or less) and the tablet of Example 6 containing non-micronized dimethyl fumarate were evaluated at pH 6.8. In addition, the elution rate evaluation was performed in the same manner as described in Example 2-1. The results are shown in Table 10 and Figure 6.

Table 9

| | D10 | D50 | D90 |
|---|---|---|---|
| Example 5 | Less than 20 μm | Less than 50 μm | Less than 100 μm |
| Example 6 | More than 20 μm | More than 50 μm | More than 100 μm |

Table 10

| Elution rate (%) | | |
|---|---|---|
| **Min.** | **Example 5** | **Example 6** |
| 0 | 0.0 | 0.0 |
| 5 | 83.3 | 16.0 |
| 10 | 93.8 | 30.9 |
| 15 | 94.9 | 44.4 |
| 30 | 95.1 | 69.1 |
| 45 | 94.8 | 79.6 |
| 60 | 94.6 | 84.5 |
| 90 | 93.5 | 88.2 |
| 120 | 92.2 | 88.9 |

As a result, it was confirmed that the elution rate of the enteric coating tablet containing dimethyl fumarate in the pH 6.8 solution was significantly affected by the particle size from the initial to the median time points. Particularly, when D90 was greater than 100 μm (Example 6), the elution rate was decreased significantly. Therefore, it is preferable that the mean particle size of the lower 90% of the dimethyl fumarate particles (D90) be 100 μm or less for the initial rapid drug release.

**<Experimental Example 5> Evaluation of elution rate according to use of alkalinizing agent**

Since dimethyl fumarate is a drug having a strong basicity with a pKa value of -6.5, it has a characteristic that the bioabsorption rate is decreased while the ionic ratio increases according to "Henderson-Hasselbalch equation" at low pH. Therefore, in order to increase the bioabsorption rate of dimethyl fumarate, it is desirable to design the drug to be rapidly released from the formulation in the pH range of 6.5 to 6.8 in the duodenum, the known drug absorption site. Accordingly, the comparative elution pattern in the pH 6.8 solution was evaluated for the composition containing meglumine ($C_7H_{17}NO_5$), an alkalinizing agent (Example 5), the composition excluding meglumine (Example 7), and the commercially available control drug (Comparative Example 1) among the compositions of the enteric coating tablets containing 120 mg of dimethyl fumarate of Examples 1 to 7 and Comparative Example 1. The results are shown in Table

11 and Figure 7.

Table 11

| Elution rate (%) | | | |
|---|---|---|---|
| **Min.** | **Example 5** | **Example 7** | **Comparative Example 1** |
| 0 | 0.0 | 0.0 | 0.0 |
| 5 | 83.3 | 67.0 | 0.5 |
| 10 | 93.8 | 83.5 | 24.2 |
| 15 | 94.9 | 85.7 | 59.4 |
| 30 | 95.1 | 86.2 | 80.5 |
| 45 | 94.8 | 85.5 | 83.1 |
| 60 | 94.6 | 85.5 | 84.2 |
| 90 | 93.5 | 84.7 | 84.8 |
| 120 | 92.2 | 83.7 | 85.3 |

As a result, the composition of Example 5 containing alkalinizing agent showed the improved initial elution rate compared to the compositions of Example 7 and Comparative Example 1 without alkalinizing agent. Therefore, the effect of allowing the initial elution rate of the drug to be rapidly released from the tablet at pH 6.8 (artificial intestinal juice) was confirmed by containing meglumine, which is used as an alkaline solubilizer among the additives included in the enteric coating tablet of Example 5.

**<Experimental Example 6> Evaluation of elution rate of enteric coating tablet containing 120 mg of dimethyl fumarate**

The comparative elution pattern was evaluated in the pH 1.2 solution (artificial gastric juice condition, disintegrating solution 1, The Korean Pharmacopoeia) and pH 6.8 solution (artificial intestinal juice) for the enteric coating tablet containing 120 mg of dimethyl fumarate (Example 5), which showed an excellent elution rate in Experimental Example 5, and the control drug (Comparative Example 1). The results are shown in Table 12, Table 13, and Figures 8 and 9, respectively.

Table 12

| pH 1.2 solution (artificial gastric juice condition) | | |
|---|---|---|
| **Elution rate (%)** | | |
| **Min.** | **Example 5** | **Comparative Example 1** |
| 0 | 0.0 | 0.0 |
| 5 | 0.2 | 0.2 |
| 10 | 0.2 | 0.2 |
| 15 | 0.2 | 0.2 |
| 30 | 0.3 | 0.2 |
| 45 | 0.4 | 0.2 |
| 60 | 0.5 | 0.3 |
| 90 | 0.7 | 0.3 |
| 120 | 0.8 | 0.4 |

Table 13

| pH 6.8 solution (artificial intestinal juice condition) | | |
|---|---|---|
| **Elution rate (%)** | | |
| **Min.** | **Example 5** | **Comparative Example 1** |
| 0 | 0.0 | 0.0 |
| 5 | 83.3 | 0.5 |
| 10 | 93.8 | 24.2 |
| 15 | 94.9 | 59.4 |
| 30 | 95.1 | 80.5 |
| 45 | 94.8 | 83.1 |
| 60 | 94.6 | 84.2 |
| 90 | 93.5 | 84.8 |
| 120 | 92.2 | 85.3 |

As a result, under the condition of artificial gastric juice, pH 1.2, almost all of the enteric coating tablet of Example 5 and the commercially available control drug according to Comparative Example 1 did not elute. On the other hand, under the condition of artificial intestinal juice, pH 6.8, both the formulations according to Example 5 and Comparative Example 1 were eluted, while the enteric coating tablet according to Example 5 was eluted faster than the formulation of Comparative Example 1.

To confirm whether the enteric coating tablet according to Example 5 exhibited the elution pattern similar to that of the commercially available formulation of Comparative Example 1, the following experiment was performed.

**Evaluation of elution pattern similarity**

To determine the similarity of elution patterns of the tablet of Example 5 (test drug) and the formulation of Comparative Example 1 (control drug) in the pH 1.2 and pH 6.8 solutions, the similarity factor ($f_2$) was calculated and compared. The similarity factor is the logarithmic reciprocal square root transformation of the sum of squared errors, and is a value obtained by measuring the similarity in the elution rate (%) between two curves, and is derived through the following mathematical formula.

[Mathematical Formula]

$$f_2 = 50 \cdot \log\{[1+(1/n)\sum_{t=1}^{n}(R_t - T_t)^2]^{-0.5} \cdot 100\}$$

n: number of time points

$R_t$: average elution rate of control drug

$T_t$: average elution rate of test drug

At this time, the appropriate time point around the average elution rate of the control drug (Comparative Example 1) was about 85% was taken as Ta, and the elution rates at 1/4Ta, 2/4Ta, 3/4Ta, and Ta were compared. The results are shown in Table 14. As a result, in the pH 1.2 and pH 6.8 solutions, all of the formulations of Example 5 and Comparative Example 1 were pharmaceutically equivalent in drug release behavior *in vitro.*

Table 14

| Test solution | 1/4 Ta | 2/4 Ta | 3/4 Ta | Ta | $f_2$ reference value | $f_2$ result value | Judgment |
|---|---|---|---|---|---|---|---|
| pH 1.2 solution | 30 min. | 60 min. | 90 min. | 120 min. | ≥55 | 99.2 | Equivalent |
| pH 6.8 solution | 45 min. | 90 min. | 120 min. | 180 min. | ≥55 | 72.3 | Equivalent |

**<Experimental Example 7> Evaluation of elution rate of enteric coating tablet containing 240 mg of dimethyl fumarate**

Since the dose-proportional linear elimination kinetics of dimethyl fumarate has been proven from 120 mg to 360 mg, a comparative elution test is possible according to the Standard on Pharmaceutical Equivalence Study of the Ministry of Food and Drug Safety based on the dose of the active ingredient within the previously approved therapeutic dose range when developing a high-dose formulation. Therefore, in order to comparatively evaluate the elution amount according to the content of dimethyl fumarate, the elution amount of the tablets of Example 5 and Example 8 was evaluated. The tablet of Example 5 contained 120 mg of dimethyl fumarate, and the tablet of Example 8 contained 240 mg of dimethyl fumarate.

The elution rate evaluation was performed in the same manner as described in Example 2-1, and the results are shown in Table 15 and Figure 10.

Table 15

| Elution rate (%) | | |
|---|---|---|
| **Min.** | **Example 5** | **Example 8** |
| 0 | 0.0 | 0.0 |
| 5 | 83.3 | 84.3 |
| 10 | 93.8 | 93.3 |
| 15 | 94.9 | 94.9 |
| 30 | 95.1 | 95.5 |
| 45 | 94.8 | 95.3 |
| 60 | 94.6 | 94.9 |
| 90 | 93.5 | 93.9 |
| 120 | 92.2 | 92.8 |

**Evaluation of elution pattern similarity**

To determine the similarity of elution patterns of the tablets of Example 8 (test drug) and Example 5 (control drug) in the pH 6.8 solution, the similarity factor ($f_2$) was calculated and compared. Similarity evaluation was performed according to the Standard on Pharmaceutical Equivalence Study of the Ministry of Food and Drug Safety as in Experimental Example 6. The results of the similarity evaluation for the tablets of Example 5 and Example 8 are shown in Table 16. As a result, in the pH 6.8 solution, the drug release behavior of the tablet of Example 8 *in vitro* was pharmaceutically equivalent to that of the tablet of Example 5.

Table 16

| Test solution | Example | $f_2$ reference value | $f_2$ result value | Judgment |
|---|---|---|---|---|
| pH 6.8 solution | Example 8 | ≥50 | 98.6 | Equivalent |

**<Experimental Example 8> *In vivo test***

After oral administration of the formulations according to Example 11 and Comparative Examples 1 to 4 to beagle dogs, a non-clinical test was performed to examine the *in vivo* kinetics of the drug. At this time, the weight ratio of the enteric coating layer of the formulation of Example 11 was 6% based on the total weight of the core, the weight ratio of the enteric coating layer of the formulation of Comparative Example 2 was 5%, the weight ratio of the enteric coating layer of the formulation of Comparative Example 3 was 10% , and the weight ratio of the enteric coating layer of the formulation of Comparative Example 4 was 12%. Particularly, 1 enteric tablet prepared in Example 11 and Comparative Examples 2 to 4 or 1 capsule according to Comparative Example 1 was orally administered to beagle dogs, and the blood sample was collected at a set time and analyzed by LC-MSMS. The results are shown in Table 17 and Figure 11. At this time, the result values of Comparative Example 3 and Comparative Example 4 were too low to be calculated, so they are not shown in Table 17.

Table 17

| | Example 11 | Comparative Example 1 | Comparative Example 2 |
|---|---|---|---|
| AUC (nghr/mL, $_{0\text{-}36}$) | 14441.31±3009.19 | 15237.59±1375.36 | 10940.91±1357.99 |
| AUC (nghr/mL, inf) | 14539.73±3008.02 | 15517.56±1452.83 | 11073.94±1399.41 |
| Cmax (ng/mL) | 6783.72±1746.61 | 6096.64±1962.29 | 5319.22±1071.96 |
| $T_{max}$ (hr) | 1.67±1.26 | 1.83±0.29 | 2.00±1.00 |

AUC: area under the time versus plasma concentration curve

$C_{max}$: maximum plasma concentration
$T_{max}$: time to reach peak plasma concentration

As shown in Table 17 and Figure 11, it was confirmed that the enteric tablet according to Example 11 exhibited pharmacokinetic parameter similar to that of the commercial product (Comparative Example 1), and that the formulations of Comparative Examples 2 to 4, in which the weight ratio of the enteric coating layer was 5%, 10%, and 12%, respectively, based on the total weight of the core, did not exhibit efficacy similar to that of the commercial product (Comparative Example 1) *in vivo.*

Particularly, as shown in Table 17 and Figure 11, the tablet of Example 11 showed almost similar AUC and Cmax values with a difference of less than 10% from the formulation of Comparative Example 1, but was not similar with the formulation of Comparative Example 2 by more than 10% difference. In addition, the compositions of Comparative Example 3 and Comparative Example 4 showed almost no drug absorption until around 12 h. From the above results, it was confirmed that when the weight ratio of the enteric coating layer of the enteric tablet containing dimethyl fumarate as a main component was less than 6% or more than 9% based on the total weight of the core, excellent pharmacokinetic results could not be obtained.

Hereinbefore, the present invention has been described in detail through preferred preparative examples, examples, and experimental examples, but the scope of the present invention is not limited to a specific example, and should be interpreted by the appended claims. In addition, those skilled in the art will understand that many modifications and variations are possible without departing from the scope of the present invention.

## Claims

1. An enteric coating tablet comprising:

   a core containing dimethyl fumarate or a pharmaceutically acceptable salt thereof as an active ingredient; and an enteric coating layer,
   wherein the enteric coating layer is included in an amount of 6 to 9 weight parts based on 100 weight parts of the core.

2. The enteric coating tablet according to claim 1, wherein the active ingredient is included in an amount of 20 to 60 weight% based on the core.

3. The enteric coating tablet according to claim 1, wherein the active ingredient is included in an amount of 60 mg to 480 mg in the core.

4. The enteric coating tablet according to claim 1, wherein the core contains one or more pharmaceutically acceptable additives selected from the group consisting of excipients, disintegrants and lubricants.

5. The enteric coating tablet according to claim 4, wherein the excipient is included in an amount of 30 to 45 weight%, the disintegrant is included in an amount of 10 to 20 weight%, and the lubricant is included in an amount of 0.1 to 2 weight% based on the core.

6. The enteric coating tablet according to claim 1, wherein the tablet further comprises a seal-coating layer between the core and the enteric coating layer.

7. The enteric coating tablet according to claim 6, wherein the seal-coating layer comprises a cellulose-based polymer.

8. The enteric coating tablet according to claim 6, wherein the seal-coating layer is included in an amount of 1 to 3 weight parts based on 100 weight parts of the core.

9. The enteric coating tablet according to claim 1, wherein the core further comprises an alkalinizing agent.

10. The enteric coating tablet according to claim 9, wherein the weight ratio of the active ingredient and the alkalinizing agent is 12:0.5 to 12:2.

11. The enteric coating tablet according to claim 9, wherein the alkalinizing agent is included in an amount of 2 to 5 weight% based on the core.

12. The enteric coating tablet according to claim 9, wherein the alkalinizing agent is meglumine or a pharmaceutically acceptable salt thereof.

13. The enteric coating tablet according to claim 1, wherein the enteric coating layer comprises one or more enteric coating polymers selected from the group consisting of enteric acrylic acid-based copolymers selected from the group consisting of styrene acrylic acid copolymer, ethyl methacrylate methacrylate copolymer, methyl acrylate acrylate octyl methacrylate copolymer and ethyl methacrylate acrylate copolymer; enteric cellulose-based polymers selected from the group consisting of hydroxypropyl methyl cellulose acetate succinate, hydroxypropyl methyl cellulose phthalate, hydroxymethyl ethyl cellulose phthalate, cellulose acetate phthalate, cellulose acetate maleate, cellulose acetate succinate, cellulose acetate maleate, cellulose benzoate phthalate, cellulose propionate phthalate, methyl cellulose phthalate, carboxymethyl ethyl cellulose, ethylhydroxy ethyl cellulose phthalate, carboxymethyl ethyl cellulose and ethyl hydroxyethyl cellulose phthalate; enteric maleic acid-based copolymers selected from the group consisting of vinyl acetate maleic acid anhydride copolymer, styrene maleic acid anhydride copolymer, styrene maleic acid monoesterol copolymer, vinyl methyl ether maleic acid anhydride copolymer, ethylene maleic acid anhydride copolymer, vinyl butyl ether maleic acid anhydride copolymer, acrylonitrile methyl acrylate maleic acid anhydride copolymer and butyl acrylate styrene maleic acid anhydride copolymer; and enteric polyvinyl-based polymers selected from the group consisting of polyvinyl alcohol phthalate, polyvinyl acetal phthalate, polyvinyl butyrate phthalate and polyvinyl acetacetal phthalate.

14. The enteric coating tablet according to claim 1, wherein the particle size distribution of dimethyl fumarate or a pharmaceutically acceptable salt thereof satisfies one or more of the following conditions:

(a) the mean particle size of the lower 90% of the particles (D90) is 100 μm or less;
(b) the mean particle size of the lower 50% of the particles (D50) is 50 μm or less; and
(c) the mean particle size of the lower 10% of the particles (D10) is 20 μm or less.

15. The enteric coating tablet according to claim 1 or claim 6, wherein the thickness of the coating layer of the enteric coating tablet is 20 μm to 90 μm.

16. The enteric coating tablet according to claim 1, wherein the core is manufactured by direct compression.

17. The enteric coating tablet according to claim 1, wherein the tablet is used for the prevention or treatment of organ fibrosis, neurodegenerative disease, psoriasis, polyarthritis, juvenile diabetes, Hashimoto's disease, Grave's disease, systemic lupus erythematosus, Sjogren's syndrome, pernicious anemia, chronic active hepatitis, lupus-like hepatitis, rheumatoid arthritis or optic neuritis.

18. The enteric coating tablet according to claim 17, wherein the organ fibrosis is at least one selected from the group consisting of renal fibrosis, cardiac fibrosis, pancreatic fibrosis, lung fibrosis, vascular fibrosis, skin fibrosis, bone marrow fibrosis, liver fibrosis, scleroderma, cystic fibrosis, pancreatic fibrosis and intestinal fibrosis; the renal fibrosis is at least one selected from the group consisting of renal failure, diabetic nephropathy, glomerulosclerosis, renal tubular fibrosis, glomerulonephritis, chronic renal failure, acute renal injury, chronic kidney disease, end-stage renal disease and albuminuria; the liver fibrosis is at least one selected from the group consisting of cirrhosis, hepatic nephropathy, hepatic purpura, metabolic liver disease, chronic liver disease, hepatitis B virus infection, hepatitis C virus infection, hepatitis D virus infection, schistosomiasis, alcoholic liver disease, non-alcoholic fat hepatitis, obesity, diabetes, protein deficiency, coronary artery disease, auto-immune hepatitis, cystic fibrosis, alpha-1 antitrypsin

deficiency and primary biliary cirrhosis; the lung fibrosis is at least one selected from the group consisting of bronchitis, acute bronchitis, diffuse panbronchiolitis (DPB), bronchiolitis, idiopathic pulmonary fibrosis (IPF), acute interstitial pneumonia, lung transplantation, radiation-induced pulmonary fibrosis, acute respiratory distress syndrome (ARDS), chronic obstructive pulmonary disease (COPD), asthma, bronchiectasis, pulmonary tuberculosis, pneumonia, pneumoconiosis, hypersensitivity pneumonia, pulmonary edema and sarcoidosis; the skin fibrosis is at least one selected from the group consisting of scarring, hypertrophic scarring, keloid scarring, cutaneous fibrosis disorder, wound healing, delayed wound healing, psoriasis and scleroderma; and the neurodegenerative diseases is at least one selected from the group consisting of multiple sclerosis, systemic sclerosis, amyotrophic lateral sclerosis, Parkinson's disease, Huntington's disease, Alzheimer's disease, acute transverse myelitis, acute disseminated encephalomyelitis, optic neuritis, acute necrotizing retinitis, transverse myelitis, chronic progressive myelopathy, progressive multifocal leukoencephalopathy, radiation myelopathy, central pontine myelinolysis, leukodystrophy, chronic inflammatory demyelinating polyneuropathy (CIDP) and acute inflammatory demyelinating polyneuropathy (AIDP).

**19.** A method for preparing an enteric coating tablet comprising the following steps:

a step of preparing a mixture by mixing dimethyl fumarate or a pharmaceutically acceptable salt thereof, and a pharmaceutically acceptable additive;
a step of preparing a core by directly tableting the mixture; and
a step of enteric coating the core,
wherein, the enteric coating is performed with 6 to 9 weight parts of the enteric coating layer based on 100 weight parts of the core.

**20.** The method for preparing an enteric coating tablet according to claim 19, wherein the method further comprises a step of seal-coating before the step of enteric coating.

**21.** The method for preparing an enteric coating tablet according to claim 19 or claim 20, wherein the step of coating is performed at 20°C to 50°C .

FIG. 1

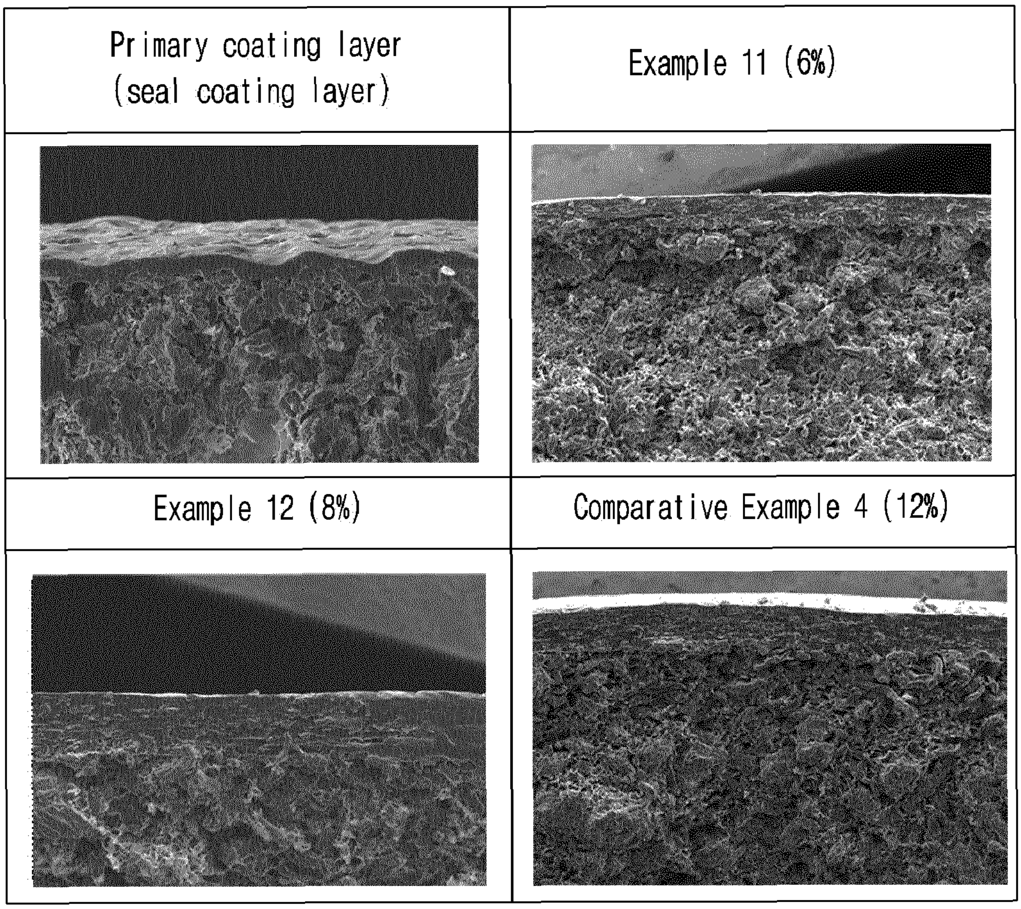
Primary coating layer
(seal coating layer)
Example 11 (6%)
Example 12 (8%)
Comparative Example 4 (12%)

FIG. 2

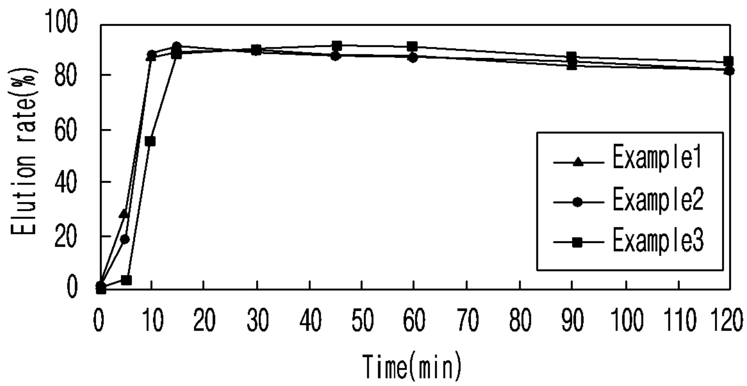
Elution rate(%)
100
80
60
40
20
0
0 10 20 30 40 50 60 70 80 90 100 110 120
Time(min)
Example1
Example2
Example3

FIG. 3

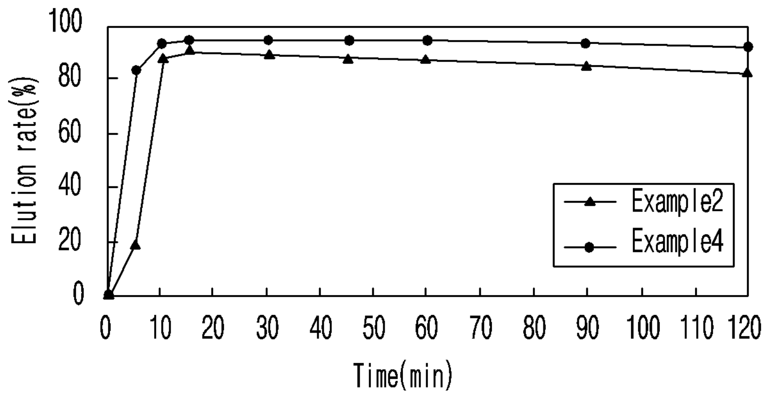
Elution rate(%)
100
80
60
40
20
0
0 10 20 30 40 50 60 70 80 90 100 110 120
Time(min)
Example2
Example4

FIG. 4

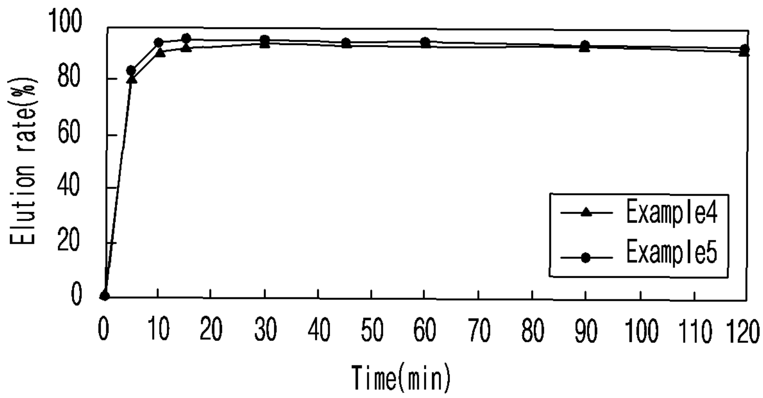
100
80
60
40
20
0
Elution rate(%)
Example4
Example5
0 10 20 30 40 50 60 70 80 90 100 110 120
Time(min)

FIG. 5

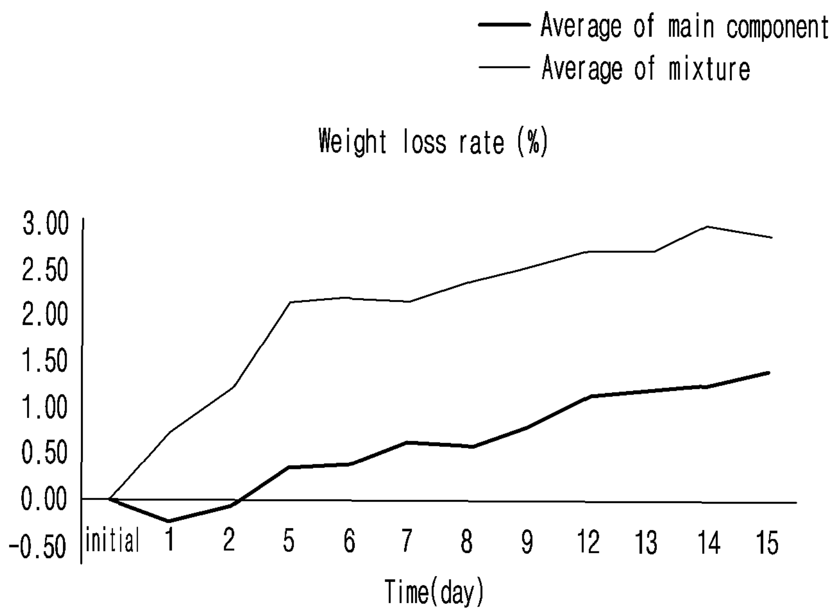
Average of main component
Average of mixture
Weight loss rate (%)
3.00
2.50
2.00
1.50
1.00
0.50
0.00
-0.50
initial 1 2 5 6 7 8 9 12 13 14 15
Time(day)

FIG. 6

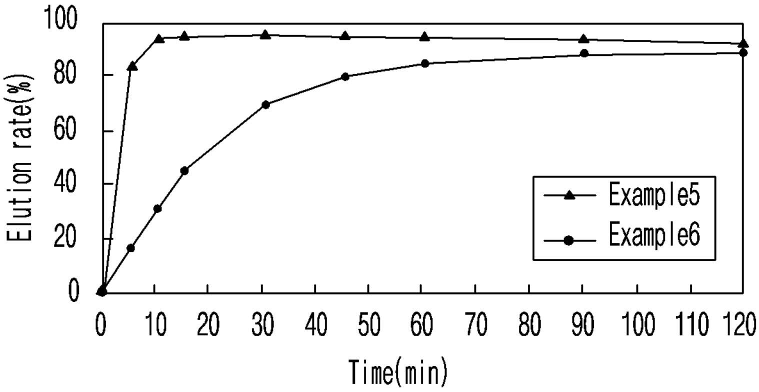
100
80
60
40
20
0
Elution rate(%)
0
10
20
30
40
50
60
70
80
90
100
110
120
Time(min)
Example5
Example6

FIG. 7

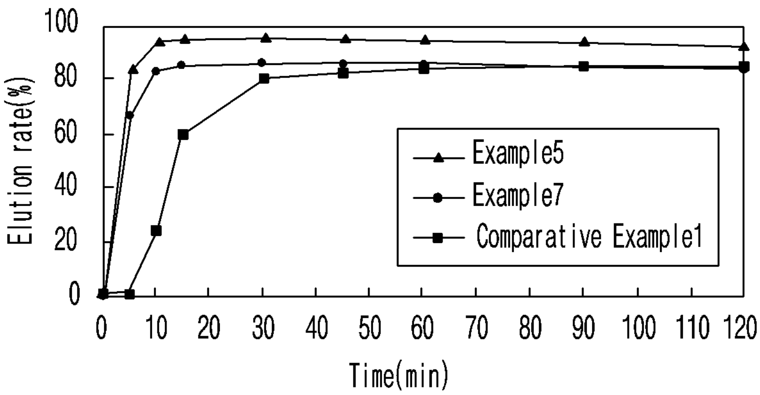
100
80
60
40
20
0
Elution rate(%)
0
10
20
30
40
50
60
70
80
90
100
110
120
Time(min)
Example5
Example7
Comparative Example1

FIG. 8

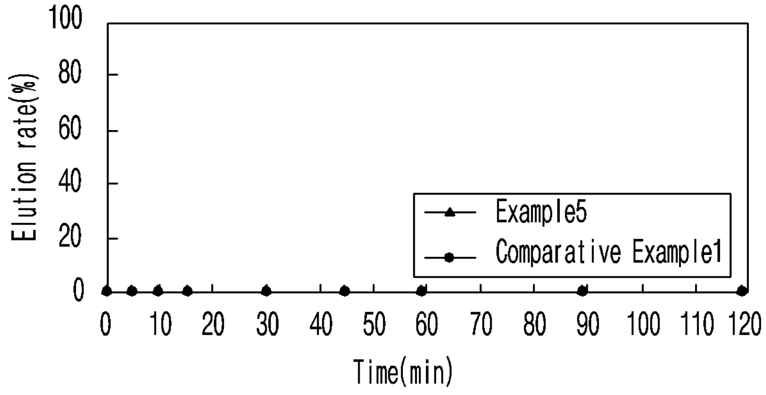
Elution rate(%)
100
80
60
40
20
0
Example5
Comparative Example1
0 10 20 30 40 50 60 70 80 90 100 110 120
Time(min)

FIG. 9

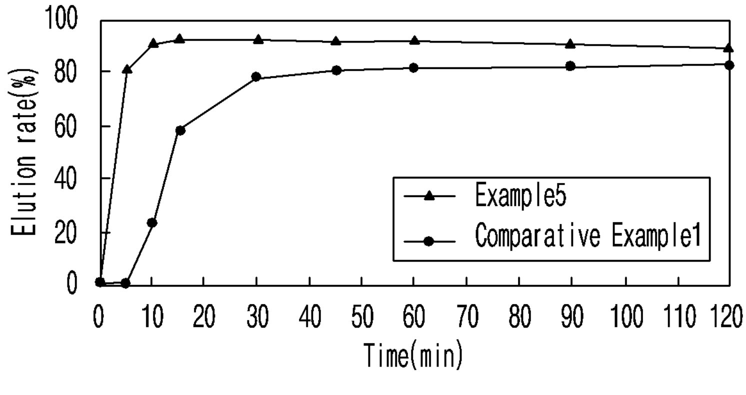
Elution rate(%)
100
80
60
40
20
0
Example5
Comparative Example1
0 10 20 30 40 50 60 70 80 90 100 110 120
Time(min)

FIG. 10

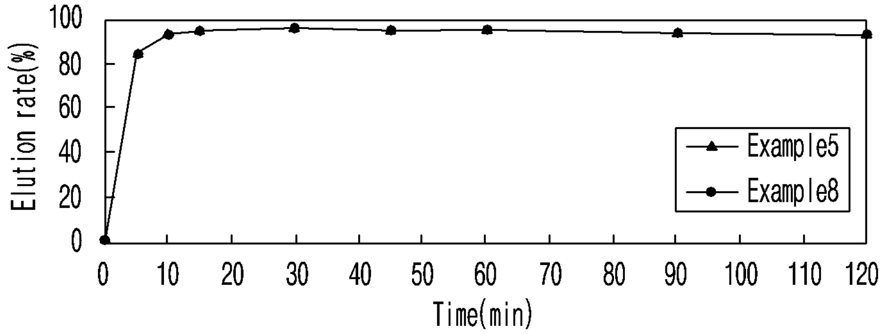
100
80
60
40
20
0
Elution rate(%)
0
10
20
30
40
50
60
70
80
90
100
110
120
Time(min)
Example5
Example8

FIG. 11

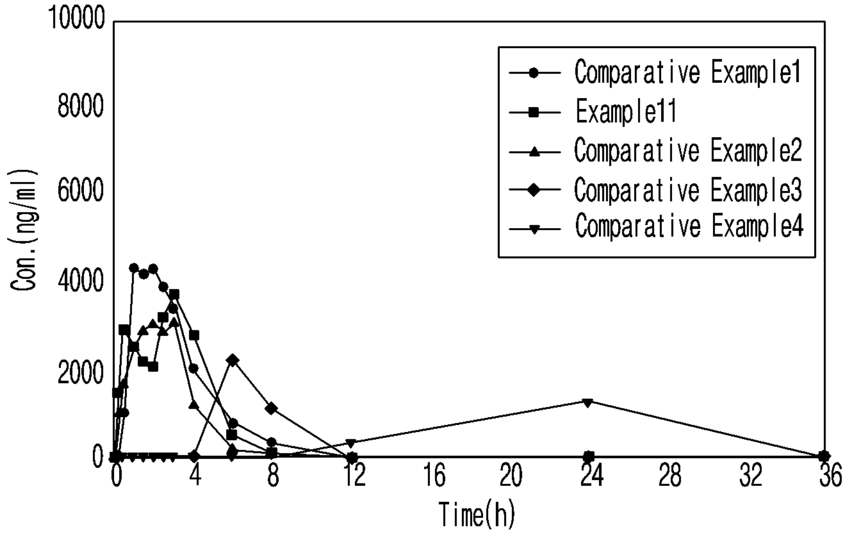
10000
8000
6000
4000
2000
0
Con.(ng/ml)
0
4
8
12
16
20
24
28
32
36
Time(h)
Comparative Example1
Example11
Comparative Example2
Comparative Example3
Comparative Example4

**INTERNATIONAL SEARCH REPORT**

International application No.
**PCT/KR2020/006647**

A. CLASSIFICATION OF SUBJECT MATTER

*A61K 9/28(2006.01)i, A61K 31/225(2006.01)i, A61P 17/06(2006.01)i, A61P 19/02(2006.01)i, A61P 43/00(2006.01)i*

According to International Patent Classification (IPC) or to both national classification and IPC

B. FIELDS SEARCHED

Minimum documentation searched (classification system followed by classification symbols)
A61K 9/28; A61K 31/225; A61K 9/24; A61P 17/06; A61P 19/02; A61P 43/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: dimethyl fumarate(DMF), enteric, coating

C. DOCUMENTS CONSIDERED TO BE RELEVANT

| Category* | Citation of document, with indication, where appropriate, of the relevant passages | Relevant to claim No. |
|---|---|---|
| X<br>Y | KR 10-2016-0076928 A (HANMI PHARM. CO., LTD.) 01 July 2016<br>See abstract; paragraphs [0033], [0064], [0071], [0121]; claims 1-19. | 1-8,13-21<br>9-12 |
| Y | KR 10-2010-0066742 A (SAMIL PHARMACEUTICAL CO., LTD.) 18 June 2010<br>See abstract; claim 1. | 9-12 |
| X | CN 104971048 A (SHANGHAI HUILUN LIFE SCIENCE & TECHNOLOGY CO., LTD.)<br>14 October 2015<br>See abstract; claims 1-11. | 1-8,13-21 |
| X | WO 2016-081676 A1 (BIOGEN MA INC.) 26 May 2016<br>See abstract; claims 1, 35-39. | 1-5,17-18 |
| X | KR 10-2016-0045728 A (FORWARD PHARMA A/S.) 27 April 2016<br>See abstract; paragraph [0282]; claims 1, 22-23. | 1-5,17-18 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 AUGUST 2020 (24.08.2020) | **01 SEPTEMBER 2020 (01.09.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

INTERNATIONAL SEARCH REPORT
Information on patent family members

International application No.
PCT/KR2020/006647

| Patent document cited in search report | Publication date | Patent family member | Publication date |
|---|---|---|---|
| KR 10-2016-0076928 A | 01/07/2016 | None | |
| KR 10-2010-0066742 A | 18/06/2010 | None | |
| WO 2016-081676 A1 | 26/05/2016 | AU 2015-349896 A1 | 25/05/2017 |
| | | CA 2967647 A1 | 26/05/2016 |
| | | CN 107205937 A | 26/09/2017 |
| | | EA 201791113 A1 | 31/10/2017 |
| | | EP 3220898 A1 | 27/09/2017 |
| | | HK 1244214 A1 | 03/08/2018 |
| | | JP 2017-534668 A | 24/11/2017 |
| | | KR 10-2017-0086063 A | 25/07/2017 |
| | | MX 2017006562 A | 19/02/2018 |
| | | US 2018-0153845 A1 | 07/06/2018 |
| KR 10-2016-0045728 A | 27/04/2016 | AU 2014-314230 A1 | 07/04/2016 |
| | | AU 2014-314231 A1 | 03/03/2016 |
| | | AU 2019-268049 A1 | 05/12/2019 |
| | | AU 2019-268052 A1 | 05/12/2019 |
| | | CA 2918846 A1 | 05/03/2015 |
| | | CA 2918852 A1 | 05/03/2015 |
| | | CN 105658207 A | 08/06/2016 |
| | | CN 105682648 A | 15/06/2016 |
| | | CN 109223725 A | 18/01/2019 |
| | | CN 109453133 A | 12/03/2019 |
| | | EA 201690102 A1 | 30/06/2016 |
| | | EA 201690107 A1 | 31/10/2016 |
| | | EP 3038605 A1 | 06/07/2016 |
| | | EP 3038606 A1 | 06/07/2016 |
| | | EP 3492072 A1 | 05/06/2019 |
| | | EP 3517102 A1 | 31/07/2019 |
| | | IL 243661 A | 29/02/2016 |
| | | IL 266015 A | 30/06/2019 |
| | | IL 266035 A | 30/06/2019 |
| | | JP 2016-528302 A | 15/09/2016 |
| | | JP 2016-531912 A | 13/10/2016 |
| | | JP 2019-189642 A | 31/10/2019 |
| | | JP 2019-196371 A | 14/11/2019 |
| | | KR 10-2016-0046813 A | 29/04/2016 |
| | | US 2016-0206586 A1 | 21/07/2016 |
| | | US 2016-0206587 A1 | 21/07/2016 |
| | | US 2017-0231943 A1 | 17/08/2017 |
| | | US 2018-0021289 A1 | 25/01/2018 |
| | | US 2018-0092875 A1 | 05/04/2018 |
| | | US 2018-0256530 A1 | 13/09/2018 |
| | | US 2018-0338946 A1 | 29/11/2018 |
| | | US 2019-0125711 A1 | 02/05/2019 |
| | | US 2019-0201369 A1 | 04/07/2019 |
| | | WO 2015-028472 A1 | 05/03/2015 |

Form PCT/ISA/210 (patent family annex) (January 2015)

**INTERNATIONAL SEARCH REPORT**
Information on patent family members

International application No.
**PCT/KR2020/006647**

| Patent document cited in search report | Publication date | Patent family member | Publication date |
|---|---|---|---|
| | | WO 2015-028473 A1 | 05/03/2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6509376 B
- US 7320999 B
- KR 20090028047
- KR 1379427
- US 6355676 B
- WO 2010126605 A


### Non-patent literature cited in the description


- **SINGH DEEP HUSSAN et al.** A review on recent advances of enteric coating. *IOSR Journal of Pharmacy,* 2012